(19) Europäisches Patentamt
European Patent Office
Office européen des brevets

(11) Publication number: **0 173 510 A2**

(12) # EUROPEAN PATENT APPLICATION

(21) Application number: **85305727.1**

(22) Date of filing: **13.08.85**

(51) Int. Cl.⁴: **C 07 K 5/06**, C 07 K 5/08, A 61 K 37/02

(30) Priority: **24.08.84 JP 176355/84**

(43) Date of publication of application: **05.03.86 Bulletin 86/10**

(84) Designated Contracting States: **CH DE FR GB IT LI**

(71) Applicant: **AJINOMOTO CO., INC., 5-8, Kyobashi 1-chome, Chuo-ku, Tokyo 104 (JP)**

(72) Inventor: **Eguchi, Chikahiko, No. 2136-10, Kamigo-cho Totsuka-ku, Yokohama-shi Kanagawa-ken (JP)**
Inventor: **Kurauchi, Masahiko, No. 5-10-36, Yatsu Nagashino-shi, Chiba-ken (JP)**
Inventor: **Sakakibara, Shumpei, No. 2-23-3, Fujishirodai, Suita-shi Osaka-fu (JP)**

(74) Representative: **Bond, Bentley George et al, Haseltine Lake & Co. 28 Southampton Buildings Chancery Lane, London WC2A 1AT (GB)**

(54) **Tripeptide derivatives for use as anti-hypertensive agents.**

(57) For use in the treatment of hypertension, the invention provides tripeptide derivatives having the general formula:

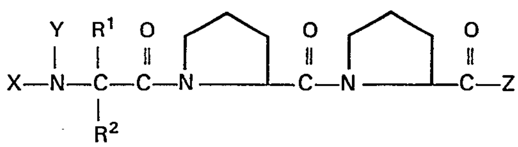

wherein X is H, R- or RCO-, Y is H-, R-, RCO-, RCS-, $RSO_2$-, $ROSO_2$-, ROCO-, RSCO-, ROCS-, RSCS-, RNHCO-, RR'NCO-, RNHCS-, RR'NCS-, RC(NR')-, $RC(NNH_2)$-, RC(NNOH)-, RNH-, RR'N-, RO-, RS-, NC-, $RNHSO_2$- or $O_2N$-, and Z is HO-, RO-, RS-, RNH-, NONH-, RONH-, $RSO_2NH$-,RCONH- or RCSNH- (wherein each of R and R' is a hydrogen atom, an alkyl group having from 1 to 6 carbon atoms, an aryl group having from 6 to 12 carbon atoms, or an aralkyl group having from 6 to 12 carbon atoms, which groups optionally contain one or more substituent groups, and wherein the group RCO- may be a residue derived from an amino acid); wherein $R^1$ and $R^2$ are the same or different and each is a hydrogen atom, an alkyl group having from 1 to 6 carbon atoms, or an aryl group having from 6 to 12 carbon atoms, or an aralkyl group having from 6 to 12 carbon atoms, which groups optionally contain one or more substituent groups, wherein, in the alternative, X and $R^1$ are combined together to form a bridge containing 2, 3 or 4 carbon atoms and optionally containing one or more hereto atoms and optionally containing one or more substituent groups: and wherein, in the alternative, X and Y are combined together to form a bridge.

EP 0 173 510 A2

# TRIPEPTIDE DERIVATIVES FOR USE AS ANTI-HYPERTENSIVE AGENTS

This invention relates to tripeptide derivatives for use as anti-hypertensive agents.

The following prior art is known to us: (1) R.S. Rapaka, D.E. Nitschi, R.S. Bhatnagar Polym: Pret, Am. Chem.Soc. Div, Polym. Chem. 16, 492 (1975) (CA, 87, 118055 w); (2) R.S. Rapaka, R.S. Bhatnagar, Int. J. Pept. Protein Res. 8,371 (1976) (CA, 85, 193100 u); (3) R.S. Rapaka, R.S. Bhatnagar, D.E. Nitecki, Biopolymers 15, 317, (1976) (CA, 84, 106063 x); (4) R. Vegners, G. Cipens, I. Dipans, Khim. Prip. Soedin. 9,763 (1973) (CA,81,152625 t); (5) Y.A. Orchinnikov, V.I. Ivanor, A.I. Miroshniko, K.K. Khalilulina, N.N. Uvarova, Khim. Prip. Soedin, 7, 469 (1971) (CA, 75, 141154 p); and (6) V.A. Shibnev, A.V. Lazareva, M.P. Finogenor, Izv. Akad. Nauk SSSR. Ser. Khim. 1969, 392 (CA, 70, 115551 t).

According to the present invention, there is provided, for use in the treatment of hypertension, a tripeptide derivative having the general formula:

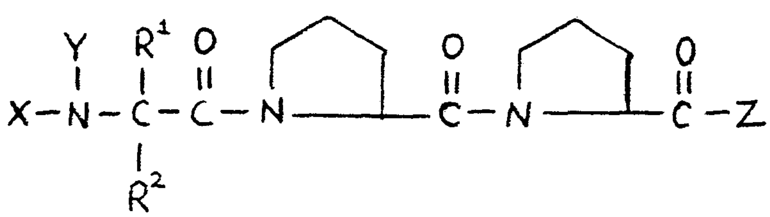

wherein X is H-, R- or RCO-, Y is H-, R-, RCO-, RCS-, $RSO_2$-, $ROSO_2$-, ROCO-, RSCO-, ROCS-, RSCS-, RNHCO-, RR'NCO-, RNHCS-, RR'NCS-, RC(NR')-, $RC(NNH_2)$-, RC(NNOH)-, RNH-, RR'N-, RO-, RS-, NC-, $RNHSO_2$ or $O_2N$- and Z is HO-, RO-, RS-, RNH-, NONH-, RONH, $RSO_2NH$-, RCONH- or RCSNH- (wherein each of R and R' is a hydrogen atom, an alkyl group having from 1 to 6 carbon atoms, an aryl group

having from 6 to 12 carbon atoms, or an aralkyl group having from 6 to 12 carbon atoms, which groups optionally contain one or more substituent groups, and wherein the group RCO- may be a residue derived from an amino acid); wherein $R^1$ and $R^2$ are the same or different and each is a hydrogen atom, an alkyl group having from 1 to 6 carbon atoms, an aryl group having from 6 to 12 carbon atoms, or an aralkyl group having from 6 to 12 carbon atoms, which groups optionally contain one or more substituents groups; wherein, in the alternative, X and $R^1$ are combined together to form a bridge containing 2, 3 or 4 carbon atoms and optionally containing one or more hereto atoms and optionally containing one or more substituent groups; and wherein, in the alternative, X and Y are combined together to form a bridge such as an alkylene bridge, an allylene bridge, or an arendiyl bridge, for example a phthaloyl bridge.

The present invention also provides a tripeptide derivative having the general formula given above wherein X, Y, $R^1$, $R^2$ and Z are as defined above, but excluding the following compounds: those wherein Y is t-butyloxcarbonyl or benxyloxycarbonyl; those wherein Y is t-amyloxycarbonyl, $R_2$ is hydrogen, X and $R^1$ are combined to form a propylene bridge, and Z is hydroxyl or benzyloxy; those wherein Y is isobutyloxy, X and $R^2$ are hydrogen, $R^1$ is sec-butyl and Z is benzyloxy; those wherein Y is triphenylmethyl, X and $R^1$ and $R^2$ are hydrogen, and Z is hydroxy; those wherein Y is o-nitrophenylsulphenyl, $R^2$ is hydrogen, X and $R^1$ are combined to form a propylene bridge, and X is hydroxy or 2,4,5-trichlorophenyloxy; those wherein Y is palmitoyl, lauroyl, 2,2-dimethylbutanoyl or isonicotinoyl, $R^2$ is hydrogen, X and $R^1$ are combined to form a propylene bridge, and Z is hydroxy; those wherein Y is acetyl, X and $R^2$ are hydrogen, $R^1$ is 1-hydroxyethyl and Z is hydroxy; those wherein Y is trifluoracetyl, X and $R^2$ are hydrogen, $R^1$ is isobutyl, and Z is methoxy; and

those wherein Y is 6-chloro-2-methoxy-9-acridinyl, X and $R^1$ are hydrogen, and Z is hydroxy.

Each of R and R' individually represents an alkyl group having from 1 to 6 carbon atoms and which optional has substituents (such as methyl, ethyl, n-propyl, n-butyl, t-butyl, cyclopentyl, cyclohexyl, carboxymethyl, carboxyethyl, aminomethyl, aminoethyl, hydroxymethyl, hydroxyethyl, mercapomethyl and mercaptoethyl); an aryl group having from 6 to 12 carbon atoms and which optionally has substituents (such as phenyl, naphthyl, p-tolyl, biphenyl, aminophenyl, hydroxy, hydroxyphenyl, and mercaptophenyl); an aralkyl group having from 6 to 12 carbon atoms and which optionally has substituents (such as benzyl, phenethyl, phenylpropyl, aminobenzyl, hydroxybenzyl and mercaptobenzyl); or an alkyl, aryl or aralkyl group which may contain a hetero ring, which has from 1 to 12 carbon atoms and which optionally has substituents (such as furyl, thienyl, pyrrolyl, pyridyl, quinolyl, thiazolyl, imidazolyl, indolyl, carbazolyl, purinyl, furfuryl, thenyl, pyridylmethyl, indolyl, pyrrodinyl, and piperadinyl).

Alternatively R and R' may be combined together to form a bridge R-R', which bridge is an alkane diyl group having from 1 to 12 carbon atoms and which optionally has substituents (such as methylene, ethylene, propane diyl, butane diyl, pentane diyl, and cyclohexane diyl) or an arene diyl group having from 1 to 12 carbon atoms and which optionally contains substituents (such as phenylene, naphthalene, naphtahalene diyl, furan diyl, piridine diyl, imidazolyl diyl, and indole diyl).

When X and/or Y is an acyl group RCO-, this acyl group may be an amino acid residue such as glycyl, alanyl, valyl, leucyl, isoleucyl, phenylalanyl, methionyl, prolyl, seryl, threonyl, arginyl, lysyl,

histidyl, glutamyl, aspartyl, glutaminyl, asparaginyl, tyrosyl, cysteinyl, tryptophyl, pyroglutamyl, or hydroxyprolyl.

The derivatives of the present invention have anti-hypersensitive activity irrespective of the nature of the substituent groups.

The amino acids constituting the derivatives of the present invention may be L-isomers or D-isomers.

The derivatives of the present invention may be in the form of a salt such as metal salt (for example the sodium, potasium, lithium or calcium salt), or a salt with an organic base. As the organic base, there can be used amines such as ammonia (ammonium salt), dicyclohexyl amine and N-methyl-D-glucamine, and basic amino acids such as lysine and arginine.

When the derivatives of the present invention are included in anti-hypertensive drugs, they are preferably in the form of a pharmaceutically acceptable salt.

The amino acid derivatives of the present invention can be prepared, for example, by reacting an alanine having a protected amino group with a proline having a protected carboxyl group to form an alanyl--proline derivative, removing the group protecting the carboxyl group, reacting the resulting product with a proline having a protected carboxyl group, and, if necessary, removing the protecting group, to form a tripeptide, namely an alanyl-prolyl-proline. The hydrogen atom in the terminal amino group threof may then be replaced by a functional group such as an acyl group.

Protecting groups for amino, imino, carboxyl and hydroxyl groups, methods for the protection of such groups, methods for the removal of the protecting groups, and methods for amino-linking by condensation between amino and carboxyl groups (as employed in the preparation of the derivatives of the present invention

and the intermediates therefor) may be those ordinarily used in the methods for peptide synthesis as generally or conventionally employed in the known literature, for example, Protein Chemistry, I, Amino Acid, Peptide, e.g. pages 405 to 509 (1969), compiled by Shiroh Akabori, Takeo Kaneko, and Kozo Narita, published by Kyoritsu Shuppan Co. An amide linkage is advantageously formed from an amino acid by a condensation method in which an active ester of the amino acid having a protected amino group, such as p-nitrophenylester or N-hydroxysuccinimideester, is used. When a solvent is used in the reaction, dimethylformamide (DMF) or water are preferred. The reaction temperature may be a room temperature or so, but the reaction can be accelerated by heating.

The derivatives of the present invention are preferably isolated from the reaction mixture by concentrating the reaction mixture to dryness, purifying the residue by means of column chromatography, followed by lyophilization of the product.

When the derivatives of the present invention are used as active ingredients for anti-hypertensive drugs, they may be used in their free forms, their nontoxic salt form, or their nontoxic forms having protecting groups. The amino acids constituting the derivatives for use as anti-hypertensive drugs of the present invention may be either L-isomers or D-isomers.

The derivatives of the present invention are useful as anti-hypertensive drugs for treating hypertension in mammals including humans. The derivatives can be used for lowering blood pressure by formulating them into preparations such as tablets, capsules, and elixirs, for oral administration and into aseptic liquid preparations or aseptic suspension preparations for parenteral administration. The derivatives of the present invention can be administered to a subject

necessitating such treatment (animals or humans) in a dosage range of 0.2 to 500 mg per subject, generally several times a day, that is, in a total daily dosage of 1 to 2000 mg. The dosage varies according to the seriousness of the disease, the body weight of the subject, and other factors known by those skilled in the art.

The derivatives of the present invention can also be administered together with diuretics or other anti-hypertensive drugs. Typically, these drugs are administered in a dosage combination of which one unit of daily dose is in the range from one-third the minimum recommended clinical dosage, to the maximum dosage recommended singly for each entity of the disease. Examples of these combinations are as-follows. An anti-hypertensive drugs of the present invention which is clinically effective in a daily dosage of 15 to 200 mg can effectively be administered together with the following other antihypersensitive drugs and diuretics in a daily dosage of 3 to 200 mg : hydrochlorothiazide (15 to 200 mg), chlorothiazide (125 to 2000 mg), ethacrynic acid (15 to 200 mg), amiloside (5 to 20 mg), furosemide (5 to 80 mg), propanolol (20 to 480 mg), timolol (5 to 50 mg), methyldopa (65 to 2000 mg). The foregoing dosage ranges are adjusted on the basis of the unit as required for the possible daily divided dosage. The dosage varies according to the seriousness of the disease, the body weight of subject, and other factors known to those skilled in the art.

The foregoing typical combinations of drugs are formulated into pharmaceutical compositions stated below. About 0.2 to 500 mg of a derivaive of the present invention, a pharmaceutically acceptable salt thereof, or a mixture of both, are blended into unit dosage forms generally acknowledged or required for

pharmaceutical practice, together with pharmaceutically acceptable vehicles, carriers, excipients, binders, antiseptics, stabilizers, flavourings, and so forth. The amount of active substance in these compositions or preparations is adjusted such as to give an appropriate dosage of the prescribed range.

Specific materials which can be incorporated into tablets, capsules, and so forth, are as follows: binders such as traganth, gum arabic, cornstarch, and gelatine; excipients such as microcrystalline cellulose; swelling agents such as cornstarch, pregelatinized starch, and arginic acid; lubricants such as magnesium stearate; sweetners such as sucrose, lactose, and sacharin, and flavourings such as peppermint, oil from Gaultheniaadeno thrix Maxim, and chery. When the unit dosage form of the preparation is in the form of a capsule, a liquid carrier such as a fatty oil can further be incorporated in the foregoing materials. Various other materials can be present as coating materials or a materials which vary the physical form of the unit dosage forms. For example, tablets can be coated with shellac and/or sugar. Syrups or elixirs can contain active compounds, sucrose as a sweetner, methyl- paraben and/or propylparaben as antiseptics, colouring matter, and a flavouring such as cherry and/or an organic flavouring agent.

Aseptic compositions for injection can be formulated according to the usual practice for preparation of pharmaceutical dosage forms, in which practice an active substance is dissolved or suspended in a vehicle such as water for injection; natural vegetable oils such as sesame oil, palm oil, peanut oil, and cotton seed oil; and synthetic fat vehicles such as ethyl oleate. A buffer, an antiseptic, and an antioxidant can further be incorporated as the occasion demands.

The invention will now be illustrated by the

following Examples, wherein "mmole" means "millimole".

Example 1 - Synthesis of N-ethoxycarbonyl-L-alanyl-L-prolyl-L-proline

Water (10 ml) and acetone (30 ml) were added to L-alanyl-L-prolyl-L-proline (5.68g, 20 mmole), and the aqueous solution thus obtained was adjusted to a pH of 12.0 with 5N aqueous sodium hydroxide. A solution obtained by mixing 90% chloroformic acid ethyl ester (2.92g, 20 mmole) with 1,2-dichloroethane (5 ml) and 5Naqueous sodium hydroxide was added dropwise at the same time over a period of 30 minutes to the above solution while stirring and while keeping the internal temperature below 10°C and the pH to 12 to 12.5. After the completion of the addition, the solution thus obtained was stirred for 1 hour at the same temperature, and then 1,2-dichloroethane (20 ml) and water (20 ml) were added thereto to result in the formation of two phases, namely an aqueous phase and a 1,2-dihcloroethane phase. To the aqueous phase, 6N hydrochloric acid was added to adjust the pH to 3.0, and then sodium chloride crystals were added thereto to change the solution to a white turbid mixture. The mixture was extracted with three 30 ml portions of chloroform. The chloroform solution was dried over anhydrous sodium sulphate, and then the chloroform was removed from the solution by distillation under reduced pressure. To the residue thus concentrated, carbon tetrachloride (30 ml) was added, and the solution was concentrated under reduced pressure. Ethyl ether (100 ml) was added to the residue thus obtained, and the mixture was allowed to solidify slowly while stirring. The solid material obtined by filtering and drying was N-ethoxycarbonyl-L--alanyl-L-prolyl-L-proline (5.7 g).

NMR spectrum [$CDCl_3$, internal standard : TMS]

1.07 to 1.45 ppm (m, 6H)    1.63 to 2.40 ppm (m, 8H)

3.20 to 3.81 ppm (m, 4H)    4.00 ppm (q, 2H)

4.17 to 4.68 ppm (m, 3H)    5.45 ppm (d, 1H)
9.00 ppm (S, 1H)

TLC [ethyl acetate;methanol = 1: 1;iodine] Rf =0.32
Mass spectrum [FAB mode]    M + H = 356

Example 2- Synthesis of N-(3-phenylpropionyl)-L alanyl-L-propyl-L-proline

(a) N-(3-phenylpropionyl)-L-alanyl-L-proplyl-L-proline

Phenylpropionic acid (3 g, 20 mmole) was dissolved in 1,2-dichlorethane (80 ml) and to the solution, while maintaining its internal temperature at 5 to 8°C, L-alanyl-L-propyl-L-proline methyl ester hydrochloride (6.69 g) triethylamine (2.02g, 20mmole), 1-hydroxybenzotriazole (0.35g, 3mmole), and dicyclohexylcarbodiimide (4.12 g, 20 mmole) were added, and the mixture thus obtained was stirred for 4 hours. Thereafter, it was stirred at room temperature overnight, and the thus precipitated dicyclohexylurea crystald and tryethylamine hydrochloride crystals were filtered off and washed with 1,2-dichloroethane (20 ml). The filtrate and the washing liquid were mixed together, and the mixture thus obtained was washed with 0.5N hydrochloric acid (50 ml), water (80 ml), 0.5N aqueous sodium hydroxide (60 ml) and water (80 ml) in the order given, and the 1,2-dichloroethane phase was concentrated under reduced pressure. To the thus obtained syrupy residue, water (50 ml) and 5N aqueous sodium hydroxide (5 ml) were added, and the thus obtained mixture was stirred at room temperature for 2 hours. After completion of the saponification, the precipitated dicyclohexylurea crystals were filtered off and washed with water (5 ml). The filtrate and the washing liquid were mixed together, and the mixture thus obtained was adjusted to a pH of 2.5 with 6N hydrochloric acid and was extracted with 1,2-dichloroethane (100 ml). The

1,2-dichloroethane phase was washed with water (30 ml), and the 1,2-dichloroethane was distilled off therefrom under reduced pressure. To the concentrated residue, carbon tetrachloride (30 ml) was added, the solution thus obtained was concentrated under reduced pressure and ethyl ether (100 ml) was added thereto. It changed slowly to a solid material while stirring. The solid material obtained by filtration and drying was N-(3-phenylpropionyl)-L-alanyl-L-prolyl-L-proline (5.8 g).

NMR spectrum [$CDCl_3$, internal standard ; TMS]

| | |
|---|---|
| 1.27 ppm (d, 3H) | 1.72 to 2.31 ppm (m, 8H) |
| 2.32 to 2.62 ppm (m, 2H) | 2.70 to 3.02 ppm (m, 2H) |
| 3.27 to 3.88 ppm (m, 4H) | 4.32 to 4.75 ppm (m, 3H) |
| 6.52 ppm (d, 1H) | 7.05 ppm (S,5H) |
| 8.99 ppm (S, 1H) | |

TLC [ethyl acetate : methanol = 1:1 ;iodine] Rf = 0.30

Mass spectrum [FAB mode] M + H = 416

(b) N-(3-phenylpropionyl)-L-alanyl-L--prolyl-L-proline L-arginine salt

N-(3-phenylpropionyl)-L-alanyl-L-prolyl-L-proline (1.00g, 2.41 mmole) was dissolved in a solution of L-arginine (0.42g, 2.41 mmole) in water (30 ml), and the thus obtained solution was freeze-dried to give N-(3-phenylpropionyl)-L-alanyl-L-prolyl-L-proline L-arginine salt (1.40g).

Example 3 - Synthesis of N-acetyl-L-alanyl-L-prolyl-L-proline

L-alanyl-L-prolyl-L-proline benzyl ester hydrochloride (2.46 g, 6.0 mmole) was dissolved in methylene dichloride (20 ml), and triethylamine (1.34 g, 13.2 mmole) was added thereto while cooling to -10°C. A solution of acetic acid anhydride (0.67 g, 6.6 mmole) in methylene dichloride (10 ml) was added thereto while

cooling to -10°C. After stirring overnight at room temperature, methylene dichloride (130 ml) was added thereto, and the thus obtained mixture was washed with 1N hydrochloric acid 9100 ml), water (100 ml) 5% aqueous sodium bicarbonae (100 ml), and water (100 ml) in the order given. The methylene dichloride phase was dried over anhydrous sodium sulphate, and the solvent was distilled off under reduced pressure to give an oily residue (2.48 g).

The oily residue was dissolved in 5% aqueous methanol (30 ml), and 5% palladium-carbon (0.5 g) was added thereto as a caalyst, and then hydrogen was passed through the solution for 2 hours. The catalyst was separated by filtration and the solvent was distilled off under reduced pressure to give a glassy residue. This residue was recrystallized from methanol-ether to obtain N-acetyl-L-alanyl-L-prolyl-L-proline (1.70g).

NMR spectrum [$CDCl_3$, internal standard : TMS]

| | |
|---|---|
| 1.33 ppm (d, 3H) | 1.70 to 2.45 ppm (m, 8H) |
| 1.96 ppm (S, 3H) | 3.27 to 3.92 ppm (m, 4H) |
| 4.40 to 4.89 ppm (m, 3H) | 5.88 to 6.88 ppm (br,2H) |

TLC [ethyl acetate : methanol = 1:1 ; iodine ] Rf=0.2

Mass spectrum [FAB mode] M + H = 326

Example 4 - Synthesis of N-propionyl-L-alanyl-L-propyl-L-proline

L-alanyl-L-prolyl-L-proline benzyl ester hydrochloride (2.46g,6.0mmole)was dissolved in methylene dichloride (20 ml), triethylamine (1.34g, 13.2 mmole) was added thereto while cooling at -10°C, and a solution of propionic acid anhydride (0.85g, 6.6 mmole) in methylene dichloride (10 ml) was added dropwise thereto at a temperature of -10°C. The mixture thus obtained was stirred overnight at room temperature and

methylene dichloride (130 ml) was added thereto, and the mixture was then washed with 1 N hydrochloric acid (100 ml), water (100 ml), 5% aqueous sodium bicarbonate (100 ml) and water (100 ml). The methylene dichloride phase was dried over anhydrous sodium sulphate, and the solvent was distilled off under reduced pressure to give an oily residue
(2.58 g).

The oily residue thus obtained was dissolved in 50% aqueous methanol (30 ml) and hydrogen was passed through the solution in the presence of 5% palladium--carbon (0.5g) as a catalyst for 2 hours. The catalyst was separated by filtration and the solvent was distilled off under reduced pressure to give a glassy residue. The residue was recrystallized from methanol--ether to obtain N-propionyl-L-alanyl-L-prolyl-L-proline crystals (1.71 g)

NMR spectrum [$CDCl_3$, internal standard : TMS]

| | |
|---|---|
| 1.13 ppm (t, 3H) | 1.33 ppm (d, 3H) |
| 1.72 to 2.45 ppm (m, 10H) | 3.35 to 3.94 ppm (m, 4H) |
| 4.45 to 4.90 ppm (m, 3H) | 5.73 to 6.90 ppm (b ,2H) |

TLC [ethyl acetate : methanol = 1:1 ;iodine] Rf =0.34

Mass spectrum [FAB mode] M + H = 340

Example 5 - Synthesis of N-picolinoyl-L-alanyl-L--prolyl-L-proline

L-alanyl-L-prolyl-L-proline benzyl ester hydrochloride (2.05 g, 5 mmole) was dissolved in chloroform (20 ml), and triethylamine (0.51g, 5 mmole), 1-hydroxybenzotriazole (0.67 g, 5 mmole), picolic acid (0.73 g, 6.0 mmole) and 1-(3-dimethylaminopropyl)-3-ethylcarbodiimide hydrochloride (0.96 g, 5 mmole) were added thereto while cooling to -15°C, and the mixture thus obtained was stirred for 1 hour at a temperature of less than 0°C and overnight at room temperature. Chloroform (100 ml) was added to the solution, and the mixture thus obtained was washed with 5% aqueous sodium

bicarbonate (100 ml) and water (100 ml) in the order given. The chloroform phase was dried over anhydrous sodium sulphate and the solvent was distilled off under reduced pressure to give an oily residue (2.36 g). The residue was purified by column chromatography (silica gel, developing solvent ethyl acetate : methanol = 20 :1) to give N-picolinoyl-L-alanyl-L--prolyl-L-proline benzylester (1.70 g). This product was dissolved in methanol (30 ml) and hydrogen gas was passed through the solution for 3 hours in the presence of 5% palladium-carbon (0.5 g) as a catalyst. The catalyst was separated by filtration, and the solvent was distilled off under reduced pressure to give a glassy residue. The residue was recrystallized from methanol-ether-petroleum ether to give N-picolinoyl-L-alanyl-L-prolyl-L-proline (1.10 g).

NMR spectrum [$CDCl_3$, internal standard : TMS]

| | |
|---|---|
| 1.50 ppm (d, 3H) | 1.80 to 2.43 ppm (m, 8H) |
| 3.35 to 4.00 ppm (m, 4H) | 4.46 to 4.79 ppm (m, 2H) |
| 4.95 ppm (f, 1H) | 6.85 to 7.45 ppm (m, 2H) |
| 7.75 ppm (t, 1H) | 8.08 ppm (d, 1H) |
| 8.50 ppm (d, 1H) | 8.63 ppm (d, 1H) |

TLC [ethyl acetate ; methanol = 1:1 ; iodine] Rf=0.23

Mass spectrum [FAB mode] M + H = 389

Example 6 - Synthesis of N-(2-thenoyl)-L-alanyl-L-proly-L-proline

L-alanyl-L-prolyl-L-proline (2.83 g, 10 mmole) was disssolved in water-dioxane (2:1, 30 ml) and the solution was adjusted to a pH of 10 with 1N aqueous sodium hydroxide. A solution of 2-thenoyl chloride (1.47 g, 10 mmole) in dioxane (10 ml) was added dropwise thereto while stirring and cooling with ice while maintaining a pH value of 9.5 to 10 with 1N aqueous sodium hydroxide. Thereafter, the mixture was stirred for 3 hours at room temperature. To this solution,

water (30 ml) and ethyl acetate (150 ml) were added and the solution was adjusted to a pH of 2 under vigorous agitation. An aqueous phase and an ethyl acetate phase were formed, and the aqueous phase was extracted again with ethyl acetate (100 ml). The ethyl acetate phase as previously separated was mixed with the ethyl acetate as next extracted, and the solution thus obtained was dried over anhydrous sodium sulphate. From the solution, the solvent was distilled off under reduced pressure to give a glassy residue (3.17 g). This product was recrystallized from ethyl acetate-carbon tetrachloride to obtain N-(2-thenoyl)-L-alanyl-L-propyl-L-proline (1.18 g).

NMR spectrum [$CDCl_3$, internal standard ; TMS]

| | |
|---|---|
| 1.44 ppm (d, 3H) | 1.70 to 2.45 ppm (m, 8H) |
| 3.35 to 4.00 ppm (m, 4H) | 4.40 to 4.73 ppm (m, 2H) |
| 4.83 ppm (f, 1H) | 6.96 ppm (t, 1H) |
| 7.23 ppm (d, 1H) | 7.33 to 7.60 ppm (m, 2H) |
| 7.88 ppm (S, 1H) | |

TLC [ethyl acetate ; methanol = 1:1; iodine] Rf = 0.31

Mass spectrum [FAB mode] M + H = 394

Example 7 - Synthesis of N-(2-furoyl)-L-alanyl-L-propyl -L-proline

L-alanyl-L-prolyl-L-proline (2.83 g, 10 mmole) was dissolved in water-dioxane (2:1. 30 ml) and the solution was adjusted to a pH of 10 with 1N aqueous sodium hydroxide. A solution of 2-furoylchloride (1.31g, 10 mmole) in dioxane (10 ml) was added dropwise thereto while stirring and cooling with ice while maintining a pH value of 9.5 to 10 with 1N aqueous sodium hydroxide, and then the mixture was stirred for 3 hours at room temperature. The solution was adjusted to a pH of 2 with 1N hydrochloric acid, concentrated under reduced pressure, and extracted with two 30 ml portions of ethyl acetate. The thus obtained ethyl

acetate phase was dried over anhydrous sodium sulphate and the solvent was distilled off under reduced pressure to give a glassy residue (2.47 g). This product was recrystallized from ethyl acetate to give N-(2-furoyl)-L-alanyl-L-prolyl-L-proline (1.48 g).

NMR spectrum [$CDCl_3$, internal standard ; TMS]

| | |
|---|---|
| 1.45 ppm (d, 3H) | 1.76 to 2.45 ppm (m,8H) |
| 3.37 to 3.97 ppm (m, 4H) | 4.45 to 4.73 ppm (m,2H) |
| 4.84 ppm (f, 1H) | 6.39 ppm (f, 1H) |
| 7.02 ppm (d, 1H) | 7.20 ppm (d, 1H) |
| 7.36 ppm (S, 1H) | 8.14 to 9.03 ppm (br,1H |

TLC [ethyl acetate : methanol = 1:1; iodine] Rf = 0.26

Mass spectrum [FAB mode] M + H = 378

Example 8 - Synthesis of N-phthaloyl-L-alanyl-L-prolyl--L-proline

L-alanyl-L-prolyl-L-proline (2.83g, 10 mmole) was dissolved in water (20 ml) in which sodium carbonate (1.06, 10 mmole) had been dissolved. To the solution, dioxane (10 ml) was added, and then a solution of N-carboethoxyphthalimide (2.19g, 10 mmole) in dioxane (10 ml) was added dropwise. The solution was stirred for 2 hours at room temperature and washed with ethyl acetate (50 ml). To the solution, water (50 ml) and ethyl acetate (150 ml) were added and the solution was adjusted to a pH of 2 with 1N hydrochloric acid while stirring vigorously. An ethyl acetate phase was separated, dried over anhydrous sodium sulphonate and the solvent was distilled off under reduced pressure to give a residue. The residue was recrystallized from ethyl acetate-ether to give N-phthaloyl-L-alanyl-L--prolyl-L-proline (1.01 g).

NMR spectrum [$CDCl_3$, internal standard ; TMS]

| | |
|---|---|
| 1.68 ppm (d, 3H) | 1.70 to 2.46 ppm (m, 8H) |
| 3.12 to 3.90 ppm (m, 4H) | 4.50 to 4.68 ppm (m, 2H) |
| 4.99 ppm (f, 1H) | 6.65 to 7.35 ppm (br, 1H) |
| 7.52 to 7.86 ppm (m, 4H) | |

TLC [ethyl acetate : methanol = 1:1; iodine ] Rf = 0.34

Mass spectrum [FAB mode] M + H = 414

Example 9 - Synthesis of N-(benzylthio)thiocarbonyl-L-alanyl-L-propyl-L-proline-L-arginine salt

(a) N-(benzylthio) thiocarbonyl-L-alanyl-L-propyl-L--proline

L-alanyl-L-prolyl-L-proline (2.83 g, 10 mmole) was dissolved in water-dioxane (2:1, 15 ml), and triethyl amine (2.13 g, 21 mmole) was added thereto. To the solution, a solution of carbon disulfide (0.76 g, 10 mmole) in dioxane (5 ml) was added dropwise. After completion of the addition, the solution was stirred for 1 hour at room temperature. Next, benzyl bromide (1.71 g, 10 mmole) was added thereto, and the mixture was stirred overnight at room temperature, and then water (100 ml) and ethyl acetate (150 ml) were added thereto. The solution was adjusted to a pH of 2 with 1N hydrochloric acid while stirring vigorously. The ethyl acetate phase was separated and dried over anhydrous sodium sulphate, and then the solvent was distilled off under reduced pressure to give a glassy residue. The residue was recrystallized from ethyl--acetate-n-hexane to give N-(benzylthio) thiocarbonyl-L-alanyl-L-propyl-L-proline (2.33 g).

NMR spectrum [$CDCl_3$, internal standard : TMS

1.47 ppm (d, 3H) 1.75 to 2.40 ppm (m, 8H)

3.30 to 3.97 ppm (m, 4H) 4.33 to 4.71 ppm (m, 2H)

4.42 ppm (S, 2H) 5.53 ppm (t, 1H)

6.64 to 7.25 ppm (br,1H)

7.99 ppm (d, 1H) 7.22 ppm (S, 5H)

TLC [methanol ; acetic acid = 20:1; spraying of 25% hydrobromic acid acetic acid solution and ninhydrine, and heating]

Rf = 0.76

Mass spectrum [FAB mode] M + H = 450

(b) N-(benzylthio) thiocarbonyl-L-alanyl-L-prolyl-L-proline L-arginine salt

N-(benzylthio) thiocarbonyl-L-alanyl-L-prolyl-L-proline(0.90 g, 2 mmole) was dissolved in a solution of L-arginine (0.35 g, 2 mmole) in water (30 ml), and the thus obtained solution was freeze-dried to give N-(benzylthio) thiocarbonyl-L-alanyl-L-prolyl-L-proline-L-arginine salt (1.22 g).

Example 10 - Synthesis of N-(3-carboxypropanoyl)-L-alanyl-L-prolyl-L-proline

L-alanyl-L-prolyl-L-proline methylester hydrochloride (3.34 g, 10 mmole) was dissolved in methylene dichloride (30 ml), and triethylamine (2.23 g, 22 mmole) was added thereto while cooling to -10°C. A solution of ethylsuccinoylchloride (1.98 g, 12 mmole) in methylene dichloride (10 ml) was added dropwise thereto while cooling to -10°C. The mixture was stirred overnight at room temperature and methylene dichloride (100 ml) was added thereto. The solution was washed with 5% aqueous sodium bicarbonate (50 ml), water (50 ml), 1N hydrochloric acid (50 ml) and water (50 ml) in the order given. The methylene dichloride layer was dried over anhydrous sodium sulphate and the solvent was distilled off under reduced pressure to give an oily residue (4.08 g). The thus obtained residue was dissolved in methanol (10 ml), and 1N aqueous solution sodium hydroxide (20 ml) was added thereto. The mixture was stirred for 2 hours at 35°C. The solution was passed through a column containing "Dowex 50W-X4 ($H^+$type)". The solution thus obtained was mixed with the washing liquid of the column, concentrated under reduced pressure, and freeze-dried to give N-(3-carboxy propanoyl)-L-alanyl-L-prolyl-L-proline (2.65 g).

NMR spectrum [ $CDCl_3$, internal standard ; TMS ]

1.33 ppm (d, 3H)
1.65 to 2.35 ppm (m, 8H)
2.36 to 2.70 ppm (m, 4H)
3.25 to 3.70 ppm (m, 4H)
4.22 to 4.80 ppm (m, 3H)
5.50 to 7.10 ppm (br,2H)
6.94 ppm (d, 1H)

TLC [ethyl acetate ; methanol = 1:1; iodine ] Rf = 0.10

Mass spectrum [FAB mode] M + H = 384

Example 11 - Synthesis of N-benzylsulphonyl-L-alanyl-L-prolyl-L-proline L-arginine salt

(a) N-benzylsulphonyl-L-alanyl-L-prolyl-L-proline

L-alanyl-L-prolyl-L-proline (3.00 g, 10.6 mmole) was dissolved in 1N aqueous sodium hydroxide (21.2 ml, 21.2 mmole), and a solution of benzylsulphonylchloride (2.02 g, 10.6 mmole) in ether (10 ml) was added dropwise thereto while stirring and cooling with ice over a period of 15 minutes. The thus obtained solution was further stirred for 3 hours at room temperature, and the ether layer was separated. The water layer was washed again with ether (10 ml). The aqueous layer was mixed with ethyl acetate (20 ml), and then adjusted to a pH of 1.0 with 6N hydrochloric acid while stirring.- The ethyl acetate layer was separated, and the aqueous layer was further extracted with ethyl acetate (20 ml). The ethyl acetate layers were combined and washed with 10% aqueous sodium chloride (20 ml), and dried over anhydrous sodium sulphate. The ethyl acetate was removed by distillation under reduced pressure to give a syrupy residue. Ether was added to the syrupy residue to cause crystallization. The thus produced crystals were filtered off, washed with ether, and dried under reduced pressure to give while crystals of N-benzylsulphonyl-L-alanyl-L-prolyl-L-proline (1.41 g).

NMR spectrum [ $CDCl_3$, internal standard : TMS ]

1.21 ppm (d, 3H)
1.70 to 2.45 ppm (m, 8H)
2.98 to 3.90 ppm (m, 4H)
4.17 ppm (S, 2H)
4.22 to 4.75 ppm (m, 3H)
5.63 ppm (d, 1H)

7.28 ppm (S, 5H)    9.00 ppm (S, 1H)

TLC [ethyl acetate ; methanol = 1:1; iodine ] Rf = 0.52

Mass spectrum [FAB mode] M + H = 438

(b) N-benzylsulphonyl-L-alanyl-L-prolyl-L-proline L-arginine salt

N-benzylsulphonyl-L-alanyl-L-prolyl-L-proline (1.00 g, 2.29 mmole) and L-arginine (0. 399 g, 2.29 mmole) were added to water (50 ml) and stirred for 1 hour at room temperature to give a homogeneous solution. The solution was filtered and the thus obtained filtrate was freeze-dried to obtain N-benzylsulphonyl--L-alanyl-L-prolyl-L-proline L-arginine salt (1.30 g).

Example 12 - Synthesis of N-ethanesulphonyl-L-alanyl-L--prolyl-L-proline

(a) N-ethanesulfonyl-L-alanyl-L-prolyl-L-proline benzylester

L-alanyl-L-prolyl-L-proline benzylester hydrochloride (4.09 g, 10.0 mmole) was added to methylene dichloride (40 ml) and triethylamine (2.02g, 20.0 mmole), was added thereto while stirring and cooling with ice. Thereafter, a solution of ethane sulphonylchloride (1.29, 10 mmole) in methylene dichloride (10 ml) was added dropwise over a period of 1 hour. The solution was allowed to stand overnight at room temperature, and then washed with two 20 ml portions of hydrochloric acid, 10% aqueous sodium chloride (20 ml), two 20 ml portions of 10% aqueous sodium bicarbonate and 10% aqueous sodium chloride (20 ml) in the order given. The methylene dichloride layer was dried over anhydrous sodium sulphate and the solvent was distilled of under reduced pressure to give a syrupy N-ethanesulphonyl-L-alanyl-L-prolyl-L-proline benzylester (3.85 g).

TLC [ethyl acetate : methanol = 1:1; iodine] Rf = 0.36

(b) N-ethanesulphonyl-L-alanyl-L-prolyl L-proline

N-ethanesulphonyl-L-alanyl-L-prolyl-L-proline benzylester (2.00 g, 4.29 mmole) was dissolved in a mixture of methanol (10 ml) and water (10 ml), and then hydrogen gas was passed through the solution in the presence of 5% palladium-carbon for 1.5 hours at room tempeature. "Cellite" was added to the solution and the solution was filtered. This operation was repeated. The catalyst was completely removed and the filtrate was concentrated under reduced pressure. The thus produced crystals were filtered off, washed with ethyl acetate, and then dried under reduced pressure to give white crystals of N-ethanesulphonyl-L-alanyl-L-prolyl-L-proline (1.17 g).

NMR spectrum [ $CDCl_3$, internal standard : TMS]

1.20 to 1.57 ppm (m, 5H)
1.80 to 2.45 ppm (m, 8H)
2.93 ppm (q, 2H)
3.36 to 3.95 ppm (m, 4H)
4.24 to 4.75 ppm (m, 3H)
5.72 ppm (d, 1H)
8.28 ppm (S, 1H)

TLC [ethyl acetate ; methanol= 1:1; iodine] Rf = 0.36

Mass spectrum [FAB mode] M + H = 376

Example 13 - Synthesis of N-benzoyl-L-alanyl-L-prolyl-L-proline

L-alanyl-L-prolyl-L-proline (3.00 g, 10.6 mmole) was dissolved in 1N aqueous sodium hydroxide (21.2 ml, 21.2 mmole), and a homogeneous solution of benzoylchloride (1.49 g, 10.6 mmole) in ether (10 ml) was added dropwise thereto over a period of 30 minutes while stirring and cooling with ice. The thus obtained solution, was stirred for 4 hours at room temperature and the solution, having a pH of 10, was washed with two 20 ml portions of ether. The aqueous layer was adjusted to a pH of 1.0 with 6N hydrochloric acid and extracted with two 30 ml portions of ethyl acetate. The ethyl acetate layer was washed with 10% aqueous

sodium chloride and dried over anhydrous sodium sulphate. The solvent was removed by distillation under reduced pressure. To the thus obtained syrupy residue, ether was added to cause crystallization, and the thus produced crystals (yield : 2.10 g) were filtered off.

The crystals were dissolved in ethyl acetate (30 ml) and the solution was filtered. To the solution, ether was added to cause the solution to become turbid, and this mixture was left overnight in a refrigerator. The crystals thus produced were filtered off, washed with ether and petroleum ether, and dried under reduced pressure to give white crystals of N-benzoyl-L-alanyl-L-prolyl-L-proline (1.01 g).

NMR spectrum [ $CDCl_3$, internal standard : TMS ]

| | |
|---|---|
| 1.48 ppm (d, 3H) | 1.80 to 2.37 ppm (m, 8H) |
| 3.40 to 4.01 ppm (m, 4H) | 4.46 to 4.77 ppm (m, 2H) |
| 4.90 ppm (t, 1H) | 7.12 to 7.53 ppm (m, 4H) |
| 7.60 to 7.87 ppm (m, 2H) | 8.40 ppm (S, 1H) |

TLC [ethyl acetate : methanol = 1:1; iodine] Rf = 0.36

Mass spectrum [FAB mode] M + H = 388

Example 14 - Synthesis of N-phenylacetyl-L-alanyl-L-prolyl-L-proline

L-alanyl-L-prolyl-L-proline (3.00 g, 10.6 mmole) and phenylacetyl chloride (1.64g, 1.06 mmole) were reacted and the reaction solution was treated in the same manner as in the above-described preparation of N-benzoyl-L-alanyl-L-prolyl-L-proline.

The reaction products were extracted with ethyl acetate and the ethyl acetate solution (40 ml) was dried over anhydrous sodium sulphate and left for 2 days in a refrigerator to produce crystals in the form of needles. The crystals were filtered off and dried to give the required product (2.27 g). The crystals were recrystallized with ethyl acetate (200 ml) to give N-phenylacetyl-L-alanyl-L-prolyl-L-proline (1.27 g).

NMR spectrum [$CDCl_3$, internal standard : TMS]

| | |
|---|---|
| 1.29 ppm (d, 3H) | 1.78 to 2.42 ppm (m, 8H) |
| 3.37 to 3.90 ppm (m, 4H) | 3.47 ppm (S, 2H) |
| 4.39 to 4.87 ppm (m, 3H) | 6.62 ppm (d, 1H) |
| 7.19 ppm (S, 5H) | 8.39 ppm (S, 1H) |

TLC [ethyl acetate : methanol = 1:1; iodine] Rf = 0.34

Mass spectrum [FAB mode] M + H = 402

Example 15 - Synthesis of N-benzyloxycarbonyl-L-alanyl-L-prolyl-L-proline L-arginine salt

(a) N-benzyloxycarbonyl-L-alanyl-L-prolyl-L-proline

To N-benzyloxycarbonyl-L-alanyl-L-prolyl-L-proline methylester (4.31 g, 10 mmole), 1.5 N aqueous sodium hydroxide (10 ml, 15 mmole) was added, and the mixture was stirred for 2 hours at 30°C. The solution was adjusted to a pH of 2.5 with 1N hydrochloric acid, and then extracted with two 50 ml portions of 1,2-dichloroethane. The 1,2-dichloroethane layer thus obtained was concentrated under reduced pressure. The residue was recrystallized from acetone-water to obtain N-benzyloxycarbonyl-L-alanyl-L-prolyl-L-proline (3.68 g).

NMR spectrum [$CDCl_3$, internal standard : TMS]

| | |
|---|---|
| 1.33 ppm (d, 3H) | 1.72 to 2.30 ppm (m, 8H) |
| 3.30 to 3.85 ppm (m, 4H) | 4.22 to 4.72 ppm (m, 3H) |
| 5.00 ppm (S, 2H) | 5.74 ppm (d, 1H) |
| 7.27 ppm (S, 5H) | |

TLC [ethyl acetate : methanol = 1:1; spraying of 25% hydrobromic acid acetic acid solution and ninhydrine, and heating}

Rf = 0.44

Mass spectrum [FAB mode] M + H = 418

(b) N-benzyloxycarbonyl-L-alanyl-L-prolyl-L-proline L-arginine salt

N-benzyloxycarbonyl-L-alanyl-L-prolyl-L-proline (1.00 g, 2.40 mmole) was dissolved in a solution of

L-arginine (0.418g, 2.40 mmole) in water (50 ml), and the solution thus obtained was freeze-dried to give N-benzyloxycarbonyl-L-alanyl-L-prolyl-L-proline L-arginine salt (1.40 g).

Example 16 - Synthesis of N-t-butyloxycarbonyl-L-alanyl-L-prolyl-L-proline

N-t-butyloxycarbonyl-L-alanyl-L-prolyl-L-proline benzylester (5.0 g, 10.6 mmole) was dissolved in methanol (100 ml), and hydrogen gas was passed through the solution in the presence of palladium-carbon as a catalyst for 3 hours. The catalyst was removed by filtration and the solvent was distllled off under reduced pressure. The residue thus obained was dissolved in water and the solution was freeze-dried to give N-t-butyloxycarbonyl-L-alanyl-L-prolyl-L-proline (4.04g).

NMR spectrum [$CDCl_3$, internal standard : TMS]

| | |
|---|---|
| 1.33 ppm (d, 3H) | 1.42 ppm (S, 9H) |
| 1.80 to 2.42 ppm (m, 8H) | 3.40 to 3.89 ppm (m, 4H) |
| 4.30 to 4.73 ppm (m, 3H) | 5.26 ppm (d, 1H) |
| 6.22 ppm (S, 1H) | |

TLC [n-butanol : acetic acid : water = 2:1:1; spraying of 25% hydrobromic acid acetic acid solution and ninhydrine and heating]

Rf = 0.69

Mass spectrum [FAB mode] M + H = 384

Example 17 - Synthesis of N-phenylcarbamoyl-L-alanyl-L-prolyl-L-proline L-arginine salt

(a) N-phenylcarbamoyl-L-alanyl-L-prolyl-L-proline

L-alanyl-L-prolyl-L-proline (3.00 g, 10.6 mmole) was dissolved in 1N aqueous sodium hydroxide (10.6 ml, 10.6 mmole), and phenylisocyanate (1.89 g. 15.9 mmole) was gradually added thereto while stirring and cooling with ice. Thereafter, the solution was stirred overnight at room temperature. The pH of the solution was about 7, and white crystals formed in the solution. The solution was adjusted to a pH of12 with 1N aqueous sodium hydroxide, and insoluble material was filtered off. The filtrate was washed with two 50 ml portions of ether, and 6N hydrochloric acid was added thereto to adjust the pH to 1.0. The solution was extracted with two 100 ml portions of ethyl acetate in order to separate the product. The ethyl acetate layer was washed with 10% aqueous sodium chloride, dried over anhydrous sodium sulphate, and concentrated under reduced pressure. To the thus concentrated material, containing crystals, ether was added to cause further crystallization. The crystals were filtered off and dried to give N-phenylcarbamoyl-L-alanyl-L-prolyl--L-proline (2.50 g).

NMR spectrum [$CDCl_3$ + DMSO-$d_6$, internal standard : TMS]

| | |
|---|---|
| 1.30 ppm (d, 3H) | 1.67 to 2.33 ppm (m, 8H) |
| 3.30 to 3.91 ppm (m, 4H) | 4.22 to 4.73 ppm (m, 3H) |
| 6.29 ppm (d, 1H) | 6.67 to 7.35 ppm (m, 5H) |
| 8.35 ppm (S, 1H) | |

TLC [methanol : acetic acid = 9:1; iodine] Rf = 0.88

Mass spectrum [FAB mode]  M + H = 403

(b) N-phenyl carbamoyl-L-alanyl-L-prolyl-L-proline L-arginine salt

N-phenylcarbamoyl-L-alanyl-L-prolyl-L-proline (1.00g, 2.49 mmole) was dissolved in a solution of L-arginine (0.43g, 2.49 mmole) in water, and the thus obtained solution was freeze-dried to give N-phenylcarbamoyl-L-alanyl-L-prolyl-L-proline L-arginine salt (1.38g).

Example 18 - Synthesis of N-phenylthiocarbamoyl-L-alanyl-L-prolyl-L-proline L- arginine salt

(a) N-phenylaminothiocarbonyl-L-alanyl-L-prolyl-L-proline

L-Alanyl-L-prolyl-L-proline (3.00 g, 10.6 mmole) was reacted with phenylisothiocyanate (2.15 g, 15.9 mmole), and the reaction solution was treated in the same manner as in the above described preparation of N-phenylcarbamoyl-L-alanyl-L-prolyl-L-proline (Example 17).

From the solution obtained by extraction with ethyl acetate, the solvent was removed by distillation under reduced pressure, and ether was added thereto. The crystals thus produced were filtered off, washed and dried to give N-phenylthiocarbamoyl-L-alanyl-L--prolyl-L-proline (2.65 g).

NMR spectrum [$CDCl_3$; internal standard :TMS]

| | |
|---|---|
| 1.38 ppm (d, 3H) | 1.67 to 2.36 ppm (m, 8H) |
| 3.24 to 4.05 ppm (m, 4H) | 4.20 to 4.74 ppm (m, 2H) |
| 5.15 ppm (t, 1H) | 7.24 ppm (S, 5H) |
| 8.45 ppm (S, 1H) | 9.67 ppm (S, 1H) |

TLC [methanol : acetic acid = 9:1; iodine] Rf = 0.76

Mass spectrum [FAB mode] M + H = 419

(b) N-phenylthiocarbamoyl-L-alanyl-L-prolyl-L-proline L-arginine salt

The required product, namely N-phenylthiocarbamoyl-L-alanyl-L-prolyl-L-proline L-arginine salt (1.38 g) was obtained by the method described above (Example 17), using N-phenylthiocarbamoyl--L-alanyl-L-prolyl-L-proline (1.00 g, 2.49 mmole) and L-arginine (0.433 g, 2.49 mmole) as starting materials.

Example 19 - Synthesis of L-prolyl-L-alanyl-L-prolyl-L-proline

L-alanyl-L-prolyl-L-proline benzylester hydrochloride (2.64g, 6 mmole), benzyloxycarbonyl-L-proline (1.65 g, 6.6 mmole) and 1-hydroxybenzotriazole (0.9 g, 6.6 mmole) were dissolved in dimethylformamide (6 ml) and 1-(3-dimethylaminopropyl)-3-ethylcarbodiimide (1.2 ml, 6.6 mmole) was added thereto at -15°C. The mixture

BAD ORIGINAL

was stirred for 16 hours at room temperature and to the reaction solution ethyl acetate was added. The solution was washed with 1N hydrochloric acid, 5% aqueous sodium bicarbonate and water in the order given. The ethyl acetate layer was dried over anhydrous sodium sulphate and the solvent was distilled off under reduced pressure. The oily residue thus obtained was dissolved in methanol-water (4:1, 25 ml), and hydrogen gas was passed through the solution in the presence of palladium-carbon as a catalyst for 2 hours. The catalyst was separated by filtration and the solvent was distilled off under reduced pressure. The residue thus obtained was recrystallized from methanol-ether to give L-prolyl-L-alanyl-L-prolyl-L-proline (1.48 g).

Elementary analysis:

Found C 52.86%, H 7.85%, N 13.01%

Calculated C 53.01%, H 7.96%, N 13.02%

(for $C_{18}H_{28}N_4O_5.CH_3OH.H_2O$)

Amino acid analysis [hydrolysis with 6N hydrochloric acid]

Ala : Pro = 1.00 : 2.94

TLC [n-butanol : acetic acid : water = 4:1:5 (above phase); spraying of 25% by diobromic acid acetic acid solution and ninhydrine, and heating]

Rf = 0.2

Mass spectrum [FAB mode] M + H = 381

Example 20 - Synthesis of glycyl-L-alanyl-L-prolyl-L-proline

L-prolyl-L-proline benzylester hydrochloride (2.46 g, 6 mmole) was dissolved in dimethylformamide (10 ml) and triethylamine (0.48 ml, 6 mmole) was added thereto while cooling to neutralize the solution. Then, benzyloxycarbonylglycine N-hydroxysuccinimideester (2.02 g, 6.6.mole) was added thereto. The solution was stirred for 2 days after its pH had been

BAD ORIGINAL

adjusted to about 7 with triethylamine, ethyl acetate (150 ml) was added thereto and the solution was washed with 1N hydrochloric acid, 5% aqueous sodium bicarbonate and water in the order given. The ethyl acetate layer was dried over anhydrous sodium sulphate and the solvent was distilled off under reduced pressure to give a residue. The residue thus obtained was recrystallized from ethyl acetate to give N-benzyloxycarbonylglycyl-L-alanyl-L-prolyl-L-proline benzylester (1.3 g). This product (1.2 g) was dissolved in methanol (50 ml), and hydrogen gas was then passed through the solution in the presence of palladium-carbon as a catalyst for 3 hours. The catalyst was separated by filtration and the solvent was distilled off. The residue thus obtained was recrystallized from methanol-ester to give glycyl-L-alanyl-L-prolyl-L-proline (688 mg).

Elementary analysis :

Found C 49.34%, H 7.93%, N 14.73%

Calculated C 49.68%, H 7.71%, N 14.48%

(for $C_{15}H_{24}N_4O_5.CH_3OH.0.8H_2O$)

Amino acid analysis [hydrolysis with 6N hydrochloric acid]

Gly : Ala : Pro = 1.00 : 0.97 : 1.96

TLC [n-butanol : acetic acid : pyridine = 15:3:12:10; spraying of 25% hydrobromic acid acetic acid solution and ninhydrine, and heating]

Rf = 0.30

Mass spectrum [FAB mode] M + H = 341

Example 21 - Synthesis of L-pyroglutamyl-L-alanyl-L-prolyl-L-proline

L-alanyl-L-prolyl-L-proline benzylester hydrochloride (2.64g, 6 mmole) was dissolved in dimethylformamide (10 ml), and triethylamine (0.84 ml, 6 mmole) was added thereto under cooling to neutralize it. Then,

0173510

benzyloxycarbonyl-L-pyroglutamic acid N-hydroxysuccinimide ester (2.38 g, 6.6 mmole) was added thereto. The mixture was stirred for 2 days after the pH of the solution had been adjusted to about 7 with triethylamine. Then, ethyl acetate (150 ml) was added thereto. The solution was washed with 1N hydrochloric acid, 5% aqueous sodium bicarbonate and water in the order given. The ethyl acetate layer was dried over anhydrous sodium sulphate and the solvent was distilled off under reduced pressure. To the residue thus obtained, n-hexane was added to form a powder. This powder was purified by column chromatography (silica gel, developing solvent chloroform: ethanol:ethyl acetate = 5:1:5). The fractions of the main ingredient were collected together and then concentrated. To the residue, n-hexane was added to precipitate a powder. This powder was dissolved in methanol (50 ml), and hydrogen gas was then passed through the solution in the presence of palladium-carbon as a catalyst for 4 hours. The catalyst was separated by filtration and the solvent was distilled off. The residue thus obtained was recrystallized from methanol-ether to give L-pyroglutamyl-L-alanyl-L-propyl-L-proline (880 mg).

Elementary analysis:

Found C 52.04%, H 7.30%, N 12.63%

Calculated C 51.99%, H 7.00 %, N 12.76%

(for $C_{18}H_{25}N_4O_6.CH_3OH.0.75H_2O$)

Amino acid analysis [Hydrolysis with 6N hydrochloric acid]

Glu : Ala : Pro = 1.00 : 0.98 : 1.96

TLC [n-butanol : acetic acid : water = 4:1:5 (above phase); spraying of 25% hydrobromic acid acetic acid solution and ninhydrine, and heating]

Rf = 0.23

Mass spectrum [FAB mode] M + H = 395

Example 22 - Synthesis of L-phenylalanyl-L-alanyl-L-prolyl-L-proline

L-alanyl-L-prolyl-L-proline benzylester hydrochloride (2.46 g, 6 mmole) was dissolved in dimethylformamide (10 ml) and triethylamine (0.84 ml, 6 mmole) was added thereto under cooling to neutrallize it. Benzyloxycarbonyl-L-phenylalanine N-hydroxysuccinimide ester (2.62g, 6.6 mmole) was added thereto. The reaction solution was stirred for 2 days after its pH had been adjusted to about 7 with triethylamine, and then ethyl acetate (150 ml) was added thereto. The mixture was washed with 1N hydrochloric acid, 5% aqueous sodium bicarbonate, and water in the order given. The ethyl acetate layer was dried over anhydrous sodium sulphate and the solvent was distilled off under reduced pressure. The residue thus obtained was recrystallized from ethyl acetate to give N-benzyloxycarbonyl-L-phenylalanyl-L-alanyl-L-prolyl-L-proline benzylester (3.2 g). This product (3 g) was dissolved in methanol (100 ml), and hydrogen gas was passed through the solution in the presence of palladium--carbon as a catalyst for 5 hours. The catalyst was separated by filtration and the solvent was distilled off from the filtrate. The residue thus obtained was recrystallized from methanol-ether to give L-phenylalanyl-L-alanyl-L-prolyl-L-proline (1.4 g).

Amino acid analysis [hydrolysis with 6N hydrochloric acid]

Ala : Pro : Phe = 1.00 : 1.94 : 0.99

TLC [n-butanol : acetic acid : water = 4:1:5 (above phase; spraying of 25% hydrobromic acid acetic acid solution and ninhydrine, and heating]

Rf = 0.19

Mass spectrum [FAB mode] M + H = 431

BAD ORIGINAL

Example 23 - Synthesis of N-benzyl-L-alanyl-L-prolyl-L-proline

A mixture of L-alanyl-L-prolyl-L-proline (12.83 g, 9.99 mmole), benzaldehyde (5.50 g, 51.8 mmole), zeolite "3A" (20 g), and ethanol (60 ml), was stirred overnight at room temperature. A suspension of sodium borohydride (1.60 g, 42.3 mmole) in ethanol (70 ml) was added dropwise thereto, and thereafter the mixture was stirred for 10 hours. The zeolite was removed by filtration and the filtrate was concentrated under reduced pressure. To the solution, ether was added to cause crystallization. The crystals thus produced were removed by filtration, washed with there 50 ml portions of ether, then purified with the ion excxhange resin "Dowex 50W-X4", and then recrystallized from ethanol-ether to give N-benzyl-L-alanyl-L-propyl-L-proline (2.10 g).

NMR spectrum [$D_2O$, internal standard : DSS]

| | |
|---|---|
| 1.52 ppm (d, 3H) | 1.73 to 2.50 ppm (m, 8H) |
| 3.24 to 3.86 ppm (m, 4H) | 3.93 to 4.58 ppm (m, 3H) |
| 4.19 ppm (S, 2H) | 7.45 ppm (S, 5H) |

TLC [methanol : acetic acid = 9:1 ; spraying of 25% hydrobromic acid acetic acid solution and ninhydrine, and heating]

Rf = 0.43

Mass spectrum [FAB mode] M + H = 374

Example 24 - Synthesis of N,N-dimethyl-L-alanyl-L-prolyl-L-proline

L-alanyl-L-prolyl-L-proline (2.83 g, 9.99 mmole), formalin (37% aqueous solution, 6 ml), 5% palladium--carbon (2.5g) and water (50 ml) were put in an autoclave, and reaction was carried out under a pressure of hydrogen of 50 kg/cm$^2$, at room temperature for 40 hours. The catalyst was removed by filtration, and the filtrate was decoloured with active carbon, and

BAD ORIGINAL

then purified with the ion exchange resin "Dowex 50W-X4", and then freeze-dried to give N,N-dimethyl-L--alanyl-L-prolyl-L-proline (1.55 g).

NMR spectrum [$D_2O$, internal standard : TMS]

| | |
|---|---|
| 1.50 ppm (d, 3H) | 1.70 to 2.51 ppm (m, 8H) |
| 2.80 ppm (S, 6H) | 3.33 to 3.88 ppm (m, 4H) |
| 4.05 to 4.44 ppm (m, 3H) | |

TLC [methanol : acetic acid = 9:1 ; iodine] Rf = 0.23

Mass spectrum [FAB mode] M + H = 312

Examples 25 to 43

The derivatives given in the following Table 1 were prepared in a manner similar to that in which the derivatives given in Examples 2, 3 and 5 were prepared.

TABLE 1

| Ex. No. | Product | The Example whose reaction was used | Starting Materials | TLC Rf value | Mass spectrum [FAB mode] (M + H) |
|---|---|---|---|---|---|
| 25 | N-benzoyl-L-isoleucyl-L-prolyl-L-proline | 3 | L-isoleucyl-L-prolyl-benzyl ester hydrochloride, benzoylchloride N-methylmorphorine | 0.71(1) | 430 |
| 26 | N-benzoyl-L-phenylalanyl-L-prolyl-L-proline sodium salt | 3(2) | L-phenylalanyl-L-prolyl-L-proline benzylester hydrochloride, benzoylchloride, N-methylmorphorine | 0.78(3) | 486 |
| 27 | N-benzoyl-glycyl-L-prolyl-L-proline | 3 | glycyl-L-prolyl-L-proline benzyl-ester hydrochloride, benzoylchloride, N-methylmorphorine | 0.64(1) | 374 |
| 28 | N-benzoyl-L-glutamyl-L-prolyl-L-proline | 3 | γ-benzyl-L-glutamyl-L-prolyl-L-proline benzylester hydrochloride, benzoylchloride, N-methylmorphorine | 0.54(3) | 446 |
| 29 | N-benzoyl-L-seryl-L-prolyl-L-proline | 5 | L-seryl-L-prolyl-L-proline benzylester hydrochloride, benzoic acid | 0.18(4) | 404 |
| 30 | N-ethoxycarbonyl-L-isoleucyl-L-prolyl-L-proline sodium salt | 2 | L-isoleucyl-L-prolyl-L-proline benzylester hydrochloride, ethoxy-carbonylchloride, N-methylmorphorine | 0.78(3) | 442 |

TABLE 1 (continued)

| | | | | | |
|---|---|---|---|---|---|
| 31 | N-ethoxycarbonyl-L-phenylalanyl-L-prolyl-L-proline sodium salt | 2 | L-phenylalanyl-L-prolyl-L-proline benzylester hydrochloride, ethoxycarbonyl chloride, N-methylmorphorine | 0.73(3) | 454 |
| 32 | N-ethoxycarbonyl-glycyl-L-prolyl-L-proline sodium salt | 2 | glycyl-L-prolyl-L-proline benzylester hydrochloriide, ethoxycarbonylchloride, N-methylmorphorine | 0.49(3) | 364 |
| 33 | N-ethoxycarbonylglutamyl-L-prolyl-L-proline | 3 | $\gamma$-benzyl-L-glutamyl-L-prolyl-L-proline benzylester hydrochloride, ethoxycarbonyl chloride, N-methylmorphorine | 0.52(3) | 414 |
| 34 | N-ethoxycarbonyl-L-arginyl-L-prolyl-L-proline | 3 | $N^{\varepsilon}$-nitro-L-arginyl-L-prolyl-L-proline benzylester, hydrochloride, ethoxycarbonylchloride, N-methylmorphorine | 0.42(3) | 441 |
| 35 | N-ethoxycarbonyl-L-seryl-L-prolyl-L-proline | 3 | L-seryl-L-prolyl-L-proline benzylester hydrochloride, ethoxycarbonylchloride, N-methylmorphorine | 0.57(1) | 372 |
| 36 | N-ethoxycarbonyl-L-methionyl-L-propyl-L-proline sodium salt | 3(5) | L-methionyl-L-prolyl-L-proline benzylester hydrochloride, ethoxycarbonyl chloride, N-methylmorphorine | 0.63(3) | 438 |
| 37 | N-ethanesulphonyl-L-isoleucyl-L-prolyl-L-proline sodium salt | 2 | L-isoleucyl-L-prolyl-L-proline benzylester hydrochloride, ethansulfonyl chloride, triethyl amine | 0.67(6) | 462 |

TABLE 1 (continued)

| | | | | | |
|---|---|---|---|---|---|
| 38 | N-ethanesulfonyl-L-phenylalanyl-L-prolyl-L-proline | 5 | N-ethanesulfonyl-L-phenylalanine dicyclohexyl amine salt, L-prolyl-L-proline benzylester hydrochloride | 0.68(7) | 452 |
| 39 | N-ethanesulfonyl-glycyl L-prolyl-L-proline | 3 | glycyl-L-prolyl-L-proline benzylester hydrochloride, ethanesulfonylchloride, triethylamine | 0.56(7) | 362 |
| 40 | N-ethane sulfonyl-L-glutamyl-L-prolyl-L proline | 3 | γ-benzyl-L-glutamyl-L-prolyl-L-proline benzylester hydrochloride, ethansulfonyl chloride, triethyl amine | 0.59(7) | 434 |
| 41 | N-ethanesulfonyl-L-arginyl-L-prolyl-L proline | 3 | $N^{\varepsilon}$-nitro-L-arginyl-L-prolyl-L-proline benzylester hydrochloride, ethanesulfonyl chloride, triethyl amine | 0.49(6) | 461 |
| 42 | N-ethanesulfonyl-L-seryl-L-prolyl-L-proline sodium salt | 2 | L-seryl-L-prolyl-L-proline benzylester hydrochloride, ethanesulfonyl chloride, triethyl amine | 0.45(6) | 414 |
| 43 | N-ethanesulfonyl-L-methionyl-L-propyl-L-proline sodium salt | 36 | L-methionyl -L-prolyl-L-proline benzylester hydrochloride, ethanesulfonyl chloride, triethyl amine | 0.60(6) | 458 |

The numerals given in brackets in Table 1 refer to the following notes.

(1) ethyl acetate: methanol: acetic acid: water = 10 : 10: 1:1; Spraying of 25% hydrobromic acid and ninhydrine, and heating.

(2) N-benzoyl-L-phenylalanyl-L-prolyl-L-proline was obtained using the same operation as in Example 3. To N-benzoyl-L-phenylalanyl-L-prolyl-L-proline (1.20g. 2.58 mmole) and sodium bicarbonate (0.217 g, 2.58 mmole), water (40 ml) was added to neutralize it, and the solution thus obtained was freeze-dried to give N-benzoyl-L-phenylalanyl-L-prolyl-L-proline sodium salt in a yield of 1.21 g (2.50 mmole, 97%).

(3) n-butanol: acetic acid: pyridine: water = 4:1:1:2; spraying of 25% hydrobromic acid and ninhydrine, and heating.

(4) ethyl acetate: spraying of 25% hydrobromic acid and ninhydrine, and heating.

(5) N-ethoxycarbonyl-L-methionyl-L-prolyl-L--proline benzylester was obtained by carrying out the same operation as in Example 3. A mixture of N-ethoxycarbonyl-L-methionyl-L-prolyl-L-proline benzylester (2.28 g, 4.51 mmole), 1N aqueous NaOH (5.4 ml) and methanol (5 ml) was stirred for 10 hours to effect a reaction. The solvent was distilled off under reduced pressure, and to the resulting residue water (50 ml) was added. The solution thus obtained was washed three times with ethyl acetate (50 ml). The solution was adjusted to a pH of 2, in the aqueous layer thereof, with 1N hydrochloric acid, and the water was distilled off under reduced pressure. To the residue, methylene dichloride (100 ml) was added, and then insoluble material was removed by filtration. The organic layer was dried over anhydrous sodium sulphate, and the solvent was distilled off under reduced pressure to obtain N-ethoxycarbonyl-L-methionyl-L-prolyl-L-proline

(0.740 g, 1.78 mmole). Next, the same operation as given in Example 26 was employed for treating this section solution to give the required product as mentioned above in a yield of 0.766 g (1.75 mmole, 39%).

(6) n-butanol: acetic acid: water = 4:1:1:2; ammonium molybdate (24 g) and cerium sulphate (1g) were dissolved in 10% sulphonic acid (500 ml) and the aqueous solution thus obtained is called "molybdenum solution" hereinafter; spraying of the molybdenum solution and heating.

(7) ethyl acetate: methanol: acetic acid: water = 10:10:1:1; spraying of the molybdenum solution and heating.

The H-NMR data for the products of Examples 25 to 43 was determined, and the results are given in the following Table 2, wherein the solvent (internal standard) used were as follows:

(a) = $CDCl_3$(TMS)

(b) = $D_2O$(TSP).

TABLE 2

| Ex. No. | Solvent (Internal Standard) | δ/ppm |
|---|---|---|
| 25 | (a) | 0.95 to 1.32(m,8H) 1.80 to 2.40(m,9H) 3.33 to 4.10(m,4H) 4.45 to 4.88(m,3H) 7.00(d,1H) 7.18 to 7.43(m,3H) 7.60 to 7.82(m,2H) |
| 26 | (b) | 1.70 to 2.55(m,8H) 2.77 to 4.99(m,9H) 6.99 to 7.72(m,1H) |
| 27 | (a) | 1.80 to 2.45(m,8H) 3.55 to 4.70(m,8H) 6.62 (bs, 1H) 7.15 to 7.80(m,5H) |
| 28 | (a) | 1.40 to 2.62(m,12H) 3.35 to 4.05(m,4H) 4.28 to 5.18(m,3H) 7.20 to 7.45(m,3H) 7.60 to 8.00(m,5H) |
| 29 | (a) | 1.55 to 2.28(m,8H) 3.35 to 4.15(m,4H) 4.30 to 4.70(m,2H) 4.88 to 5.17(m,1H) 5.70 (bs, 1H) 5.97 (bs, 1H) 7.20 to 7.43(m,4H) 7.50 to 7.80(m,2H) |
| 30 | (b) | 0.80 to 1.10(m,8H) 1.20 (t, 3H) 1.75 to 2.40(m,7H) 3.40 to 3.95(m,4H) 4.00 to 4.30(m,3H) 4.01 (q,2H) |
| 31 | (b) | 1.00 to 1.31(m,3H) 1.75 to 2.52(m,8H) 2.60 to 4.32(m,9H) 3.90 ( q,2H) 6.97 to 7.35(m,6H) |

TABLE 2 (continued)

| | | |
|---|---|---|
| 32 | (b) | 1.25 (t,3H) 1.68 to 2.60(m,8H)<br>3.35 to 4.59(m,6H) 3.95 (S,2H)<br>4.08(q,2H) |
| 33 | (a) | 1.17 (t, 3H) 1.67 to 2.55(m,12H)<br>3.35 to 3.85(m,4H) 4.00 (d,2H)<br>4.25 to 4.65 (m,3H) 5.82 (d, 1H)<br>1.82 (bs, 1H) 8.20 (bs, 1H) |
| 34 | (b) | 1.23 (t, 3H) 1.50 to 2.50(m,12H)<br>3.05 to 4.60(m,9H) 4.05 (q,2H) |
| 35 | (a) | 1.25 (t, 3H) 1.90 to 2.44(m,8H)<br>3.55 to 4.80(m,7H) 4.07 ( q , 2H)<br>5.45 (bs, 1H) 5.55 (bs, 1H) |
| 36 | (b) | 1.20 (t, 3H) 1.60 to 2.40(m,10H)<br>2.10 (S, 3H) 2.64 ( t, 2H)<br>3.37 to 4.65(m,7H) 4.10 ( q, 2H) |
| 37 | (b) | 0.95 to 1.20(m,9H) 1.33 (t, 3H)<br>1.50 to 2.55(m,8H) 3.02 ( q, 2H)<br>3.45 to 4.37 (m,7H) |
| 38 | (b) | 0.95 to 1.40(m,3H) 2.00 to 2.60(m,8H)<br>2.75 to 3.98(m,9H) 6.41 (bs, 1H)<br>7.05 to 7.33(m,5H) |
| 39 | (a) | 1.25 to 1.50(m,3H) 1.70 to 2.60(m,8H)<br>3.02 ( q,2H) 3.30 to 3.80(m,4H)<br>3.83 (bs, 2H) 4.30 to 4.75(m,3H)<br>5.51 (bs, 1H) |

BAD ORIGINAL

TABLE 2 (continued)

| | | | |
|---|---|---|---|
| 40 | (a) | 1.20 to 1.55(m,3H) | 2.00 to 2.65(m,12H) |
| | | 2.88 ( q,2H) | 3.50 to 3.95(m,4H) |
| | | 4.90 to 5.25(m,3H) | 6.10 (d, 1H) |
| 41 | (b) | 1.20 to 1.45(m,3H) | 1.50 to 2.70(m,12H) |
| | | 2.90 (d, 2H) | 3.10 to 4.65(m,9H) |
| 42 | (b) | 1.30 (m, 3H) | 1.24 to 2.65(m,8H) |
| | | 3.23 ( q,2H) | 3.48 to 4.00(m,4H) |
| | | 3.67 (d, 2H) | 4.15 to 4.85(m,3H) |
| 43 | (b) | 1.30 (t, 3H) | 1.75 to 2.51(m,10H) |
| | | 2.11 (S, 3H) | 2.70 ( 2H) |
| | | 3.15 ( q,2H) | 3.38 to 4.81(m,7H) |

Example 44

The anti-hypertensive activity of the derivatives as prepared above was determined.

As subject animals, there were used 5 SHR male rats (bodyweight 400 - 440g) which had been trained and had been confirmed to be hypertensive, per sample.

As an instrument for measuring blood pressure, there was used a Programmed Electro-Sphygomomanometer PE-300 (Narco Co., U.S.A.). The blood pressure was indirectly measured on conscious rats.

An aqueous solution of a sample (0.05 mmole/kg) of the derivative was once force fed into the stomach of the rat by means of a peroal probe. As a control, deionized water was fed to the animal.

The results obtained are given in the following Table 3.

TABLE 3

Systolic Blood Pressure, mmHg

| Example | Value before administration | Lowest value after administration | Hours after administration | ΔBlood pressure |
|---|---|---|---|---|
| 1 | 193 | 155 | 8 | -38 |
| 2 | 190 | 154 | 24 | -36 |
| 3 | 201 | 160 | 8 | -41 |
| 4 | 197 | 173 | 8 | -24 |
| 5 | 197 | 175 | 8 | -22 |
| 6 | 202 | 182 | 8 | -20 |
| 7 | 205 | 177 | 8 | -28 |
| 8 | 225 | 207 | 4 | -18 |
| 9 | 216 | 191 | 24 | -25 |
| 11 | 202 | 174 | 8 | -28 |
| 12 | 204 | 159 | 24 | -45 |
| 13 | 220 | 177 | 8 | -43 |
| 14 | 199 | 175 | 8 | -24 |
| 15 | 205 | 172 | 4 | -33 |
| 16 | 194 | 173 | 8 | -21 |
| 17 | 218 | 203 | 24 | -15 |
| 18 | 224 | 211 | 24 | -13 |
| 21 | 219 | 190 | 8 | -29 |
| 25 | 229 | 201 | 8 | -28 |
| 26 | 228 | 194 | 8 | -34 |
| 27 | 232 | 209 | 8 | -23 |
| 28 | 235 | 196 | 8 | -39 |
| 29 | 214 | 192 | 8 | -22 |
| 30 | 224 | 208 | 4 | -16 |
| 31 | 216 | 199 | 24 | -17 |
| 32 | 215 | 193 | 8 | -22 |
| 33 | 220 | 202 | 8 | -18 |

TABLE 3 (Continued)

Systolic Blood Pressure, mmHg

| Example | Value before administration | Lowest value after administration | Hours after administration | ΔBlood pressure |
|---|---|---|---|---|
| 34 | 203 | 192 | 4 | -11 |
| 35 | 220 | 195 | 8 | -25 |
| 36 | 223 | 206 | 8 | -17 |
| 37 | 218 | 194 | 8 | -22 |
| 38 | 235 | 201 | 8 | -34 |
| 39 | 232 | 215 | 8 | -18 |
| 40 | 220 | 202 | 8 | -18 |
| 41 | 204 | 185 | 7 | -19 |
| 42 | 220 | 200 | 24 | -20 |
| 43 | 229 | 198 | 8 | -31 |

CLAIMS

1. For use in the treatment of hypertension, a tripeptide derivative having the general formula:

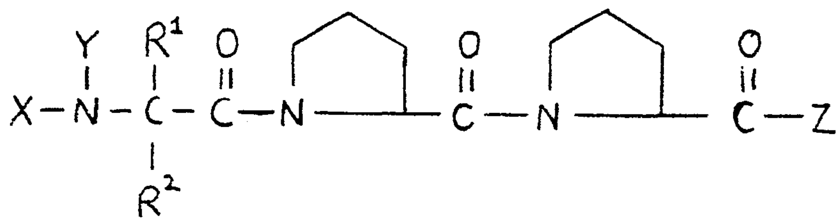

wherein X is H-, R- or RCO-, Y is H-, R-, RCO-, RCS-, $RSO_2$-, $ROSO_2$-, ROCO-, RSCO-, ROCS-, RSCS-, RNHCO-, RR'NCO-, RNHCS-, RR'NCS, RC(NR')-, $RC(NNH_2)$-, RC(NNOH)-, RNH-, RR'N-, RO-, RS-, NC-, $RNHSO_2$ or $O_2N$- and Z is HO-, RO-, RS-, RNH-, NONH-, RONH-, $RSO_2NH$-, RCONH- or RCSNH- (wherein each of R and R' is a hydrogen atom, an alkyl group having from 1 to 6 carbon atoms, an aryl group having from 6 to 12 carbon atoms, or an aralkyl group having from 6 to 12 carbon atoms, which groups optionally contain one or more substituent groups, and wherein the group RCO- may be a residue derived from an amino acid); wherein $R^1$ and $R^2$ are the same or different and each is a hydrogen atom, an alkyl group having from 1 to 6 carbon atoms, an aryl group having from 6 to 12 carbon atoms, or an aralkyl group having from 6 to 12 carbon atoms, which groups optionally contain one or more substituents groups; wherein, in the alternative, X and $R^1$ are combined together to form a bridge containing 2, 3 or 4 carbon atoms and optionally containing one or more hereto atoms and optionally containing one or more substituent groups; and wherein, in the alternative, X and Y are combined together to form a bridge.

2. For use in the treatment of hypertension, a tripeptide derivative as claimed in claim 1, wherein $R^1$ is $CH_3$- and $R^2$ is H-.

3. For use in the treatment of hypertension, a tripeptide derivative as claimed in claim 1 or 2, wherein each of R and R' (of the groups X, Y and Z) is a group selected from methyl, ethyl, n-propyl, n-butyl, phenyl, naphthyl, p-tolyl, biphenyl, benzyl, phenethyl, phenylpropyl, carboxymethyl, carboxyethyl, aminomethyl, aminoethyl, hydroxymethyl, hydroxyethyl, mercaptomethyl, mercaptoethyl, aminophenyl, aminobenzyl, hydroxyphenyl, hydroxybenzyl, mercaptophenyl, mercaptobenzyl, cyclopentyl, cyclohexyl, furyl, thienyl, pyrrolyl, pyridyl, quinolyl, thiazolyl, imidazolyl, indolyl, carbazolyl, purinyl, furfuryl, thenyl, pyridylmethyl, indolinyl and piperadinyl.

4. For use in the treatment of hypertension, a tripeptide derivative as claimed in claim 1 or 2, wherein X and/or Y is a group RCO- selected from glycyl, alanyl, valyl, leucyl, isoleucyl, phenyalanyl, methionyl, prolyl, seryl, threonyl, arginyl, lysyl, histidyl, glutamyl, aspartyl, glutaminyl, asparaginyl, tyrosyl, cysteinyl, typtophyl, pyroglutamyl and hydroprolyl.

5. For use in the treatment of hypertension, a tripeptide derivative as claimed in claim 1 or 2, wherein one or more of the amino acids constituting the derivative is in the L-form.

6. For use in the treatment of hypertension, a salt of a tripeptide derivative as claimed in any of claims 1 to 5.

7. For use in the treatment of hypertension, a salt as claimed in claim 6, being a salt selected from the sodium, potassium, lithium, calcium, ammonium, dicylcohexyl amine, N-methyl-D-glucamine and basic amino acid salts.

8. A tripeptide derivative having the general formula given in claim 1 wherein X, Y $R^1$, $R^2$ and Z are as defined in claim 1, but excluding the following

compounds: those wherein Y is t-butyloxcarbonyl or benzyloxycarbonyl; those wherein Y is t-amyloxy-carbonyl, $R^2$ is hydrogen, X and $R^1$ are combined to form a propylene bridge, and Z is hydroxy or benzyloxy; those wherein Y is isobutyloxy, X and $R^2$ are hydrogen, $R^1$ is sec-butyl and Z is benzyloxy; those wherein Y is triphenylmethyl, X and $R^1$ and $R^2$ are hydrogen, and Z is hydroxy; those wherein Y is o-nitrophenylsulphenyl, $R^2$ is hydrogen, X and $R^1$ are combined to form a propylene bridge, and Z is hydroxy or 2,4,5-trichlorophenyloxy; those wherein Y is palmitoyl, lauroyl, 2,2-dimethyl-butanoyl or isonicotinoyl, $R^2$ is hydrogen, X and $R^1$ are combined to form a propylene bridge, and Z is hydroxy; those wherein Y is acetyl, X and $R^2$ are hydrogen, $R^1$ is 1-hydroxyethyl and Z is hydroxy; those wherein Y is trifluoroacetyl, X and $R^2$ are hydrogen, $R^1$ is isobutyl, and Z is isobutyl, and Z is methoxy; and those wherein Y is 6-chloro-2-methoxy-9-acridinyl, X, $R^1$ and $R^2$ are hydrogen, and Z is hydroxy.

9. A tripeptide derivative as claimed in claim 8, wherein R is $CH_3$- and $R^2$ is H-.

10. A tripeptide as claimed in claim 8 or 9, wherein each of R and R' (of the groups, X, Y and Z) is a group selected from methyl, ethyl, n-propyl, n-butyl, phenyl, naphthyl, p-tolyl, biphenyl, benzyl, phenethyl, phenylpropyl, carboxymethyl, carboxyethyl, aminomethyl, aminoethyl, hydroxymethyl, hydroxyethyl, mercaptomethyl, mercaptoethyl, aminophenyl, aminobenzyl, hydroxyphenyl, hydroxybenzyl, mercaptophenyl, mercapto-benzyl, cyclopentyl, cyclohexyl, furyl, thienyl, pyrrolyl, pyridyl, quinolyl, thiazolyl, imidazolyl, indolyl, carbazolyl, purinyl, furfuryl, thenyl, pyridylmethyl, indolinyl and piperadinyl.

11. A tripeptide derivative as claimed in claim 8 or 9, wherein X and/or Y is a group RCO-, selected from glycyl, alanyl, valyl , leucyl, isoleuycl, phenylalanyl, methionyl, prolyl, seryl, threonyl, arginyl, lysyl, histidyl, glutamyl, aspartyl, glutaminyl, asparaginyl, tyrosyl, cysteinyl, typtophyl, pyroglutamyl and hydroprolyl.

12. A tripeptide derivative as claimed in claim 8 or 9, wehrein one or more of the amino acids constituting the derivative is in the L-form.

13. A salt of a tripeptide derivative as claimed in any of claims 8 to 12.

14. A salt as claimed in claim 13, being a salt selected from the sodium, potassium, lithium, calcium, ammonium, dicyclohexyl amine, N-methyl-D-glucamine and basic amino acid salts.

15. A drug for the treatment of hypertension, comprising (a) a tripeptide derivative as defined in any of claims 1 to 5 or a salt as defined in claim 6 or 7, and (b) a pharmaceutically-acceptable carrier or diluent.

16. A drug as claimed in claim 15, further comprising a diuretic.